# FASCICULE DE BREVET EUROPEEN

(11) **EP 0 715 836 B1**
(45) Date de publication et mention de la délivrance du brevet: **10.10.2001**
(21) Numéro de dépôt: 95402788.4
(22) Date de dépôt: 23.11.1995
(51) Int. Cl.: A61F 2/40

(54) **Endoprothèse totale d'épaule**
Totale Schultergelenk Endoprothese
Total shoulder endoprosthesis

(30) Priorité: 08.12.1994 FR 9414962
(43) Date de publication de la demande: 12.06.1996
(73) Titulaire: CEDIOR S.a.r.L, F-25461 Etupes Cedex (FR); Gerber, Christian, 1752 Villars sur Glane (CH)
(72) Inventeur: Pequignet, Yves, F-25000 Besançon (FR); Gerber Christian, F-25000 Besançon (FR)
(74) Mandataire: Ballot, Paul Denis Jacques

(56) Documents cités:
- WO-A-93/09733
- FR-A- 2 689 756
- FR-A- 2 701 206

## Description

La présente invention concerne une prothèse totale d'épaule.

Une prothèse totale selon le préambule de la revendication 1 est connue du document FR-A-2 689 756.

L'invention trouve une application particulièrement avantageuse dans le domaine du traitement des affections dégénératives de l'arthrose glèno-humérale et de la traumatologie de l'articulation de l'épaule.

D'une manière générale, on connaît des prothèse totales d'épaule comprenant une tige humérale destinée à être implantée à l'intérieur du canal huméral d'un patient, ladite tige portant une calotte sphérique de révolution, laquelle est destinée à coopérer avec la glène de l'épaule, qu'elle soit prothétique ou non.

Il existe, en particulier, des prothèses de ce type dont la tige est dite monobloc, en ce sens que la calotte sphérique humérale est incluse avec la tige elle-même. Le modèle de NEER en est le meilleur exemple.

Toutefois, outre le fait qu'elles exigent la constitution d'un stock important, ces prothèses connues présentent l'inconvénient de ne pas permettre de déport médial, ou excentration, caractérisé par le croisement de l'axe métaphysaire proximal avec la périphérie de la calotte articulaire. De plus, leur inclinaison étant fixe, elles ne recouvrent pas de manière satisfaisante le plan de coupe de l'extrémité de l'humérus. Enfin, aucune possibilité de réglage en rétroversion ne peut être envisagée avec ces prothèses monobloc.

Par ailleurs, d'autres modèles de tiges humérales permettent d'obtenir, à l'aide de cales en forme de coin placées entre la tige et la calotte, des inclinaisons de ladite calotte sphérique variant par pas de manière séquentielle. Ces mêmes tiges autorisent également un certain réglage du déport médial par rotation de la calotte sphérique autour d'un axe excentré par rapport à son axe géométrique. Cependant, là encore, les possibilités de réglage sont limitées car elles ne peuvent être effectuées que sur un nombre fini de positions discontinues. On observera en outre que, même si elles constituent un progrès par rapport aux prothèses monobloc, ces tiges humérales connues n'apportent aucune réponse à la question du réglage en rétroversion.

Aussi, le problème technique à résoudre par l'objet de la présente invention est de proposer une prothèse totale d'épaule, comprenant :
- une tige humérale destinée à être implantée à l'intérieur du canal huméral d'un patient,
- une calotte sphérique de révolution autour d'un axe, destinée à coopérer avec la glène de l'épaule,
prothèse qui serait pourvue de moyens de réglage continu, et non seulement discret, de la calotte sphérique dans toutes les orientations possibles, y compris la rétroversion, par rapport à la tige humérale, permettant ainsi de reproduire l'anatomie exacte de l'épaule.

La solution au problème technique posé consiste, selon la présente invention, en ce que ladite prothèse comporte :
- des moyens de liaison de ladite calotte à la tige humérale, comprenant une rotule sphérique fixée à une extrémité proximale de ladite tige, et une cavité sphérique aménagée dans ladite calotte et formant logement pour ladite rotule, l'axe de révolution de la calotte sphérique étant déporté par rapport au centre de ladite rotule sphérique, l'ensemble de la cavité et de la rotule constituant une articulation apte à faire varier l'orientation de la calotte sphérique relativement à la tige par rotation autour dudit centre de la rotule,
- des moyens de blocage en position de la calotte sur la rotule sphérique.

Ainsi, lesdits moyens de liaison permettent d'obtenir les trois mouvements recherchés pour la calotte sphérique, à savoir l'inclinaison, le déport médial du fait du déport de l'axe de révolution de la calotte par rapport au centre de la rotule sphérique, et la rétroversion latérale. Bien entendu, ces mouvements sont réalisés de façon parfaitement continue et dans toutes leurs combinaisons possibles, ce qui assure une reconstitution très précise de l'anatomie de l'épaule.

Selon un mode particulier de l'invention, les moyens de blocage comprennent, d'une part, au moins une fente médiane et un alésage conique axial débouchant dans ladite fente médiane, aménagés dans la rotule sphérique, et, d'autre part, un poussoir conique disposé dans ledit alésage et commandé par une vis de blocage de manière à exercer une force de pression sur l'alésage conique provoquant une compression contre la cavité sphérique par expansion latérale bloquée de ladite rotule.

La description qui va suivre en regard des dessins annexés, donnés à titre d'exemples non limitatifs, fera bien comprendre en quoi consiste l'invention et comment elle peut être réalisée.

La figure 1 est une vue en coupe partielle d'une prothèse totale d'épaule conforme à l'invention.

La figure 2 est une vue en perspective d'un mode de réalisation particulier d'une rotule sphérique permettant le blocage de la calotte de la figure 1.

La figure 3 est une vue de côté montrant la calotte de la figure 1 en position sur la rotule sphérique de la figure 2.

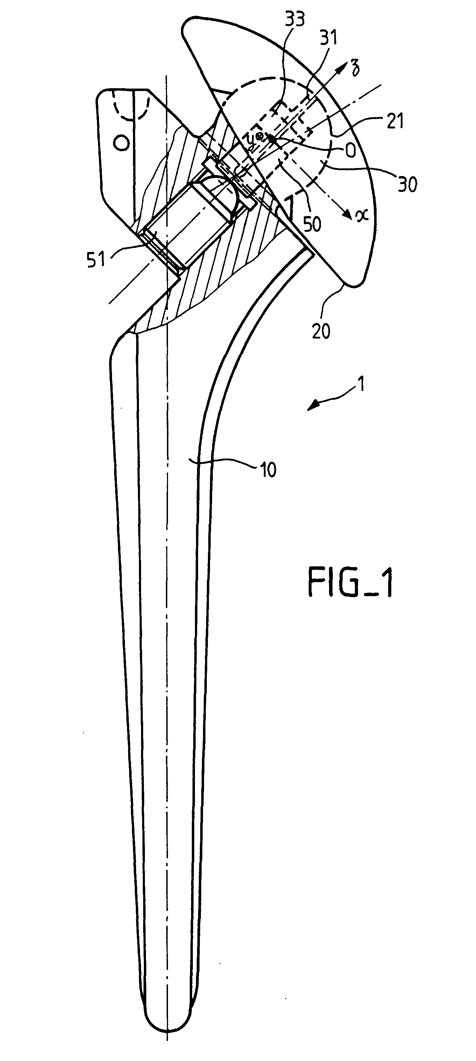

La figure 1 montre en coupe partielle une prothèse totale 1 d'épaule comprenant une tige humérale 10 destinée à être implantée à l'intérieur du canal huméral d'un patient. D'autre part, une calotte sphérique 20 présentant un axe D de révolution est destinée à coopérer avec la glène de l'épaule dudit patient.

Comme on peut le voir sur la figure 1, ladite prothèse 1 d'épaule comporte des moyens de liaison de la calotte 20 à la tige humérale 10 comprenant une rotule sphérique 30 de centre 0, fixée à une extrémité proximale de ladite tige 10, tandis qu'une cavité sphérique 21 aménagée dans ladite calotte 20 forme logement pour ladite rotule 30. On observera sur la figure 1 que l'axe D de révolution de la calotte sphérique 20 est déportée par rapport au centre 0 de la rotule sphérique 30.

Les mouvements de rotation de la calotte 20 peuvent être décomposés en trois rotations élémentaires : l'une autour d'un axe Oy transversal à la tige 10 et perpendiculaire à l'axe Oz de la rotule 30, qui permet l'inclinaison de la calotte 20, une autre autour dudit axe Oz correspondant à l'excentration, ou déport médial, de ladite calotte sphérique 20 résultant du déport de l'axe D de révolution de la calotte par rapport au centre 0 de la rotule sphérique 30, et enfin la rotation autour de l'axe Ox qui réalise la rétroversion latérale de la calotte, ce degré de liberté supplémentaire étant jusque là ignoré des prothèses d'épaule habituellement utilisées.

On notera également le caractère continu de ces trois mouvements de rotation, sans aucune notion de réglage par pas discrets comme dans les prothèse connues. Cet avantage, associé à celui de pouvoir disposer de tous les degrés de liberté en rotation, permet d'atteindre une reproduction de l'anatomie de l'épaule aussi exacte que possible.

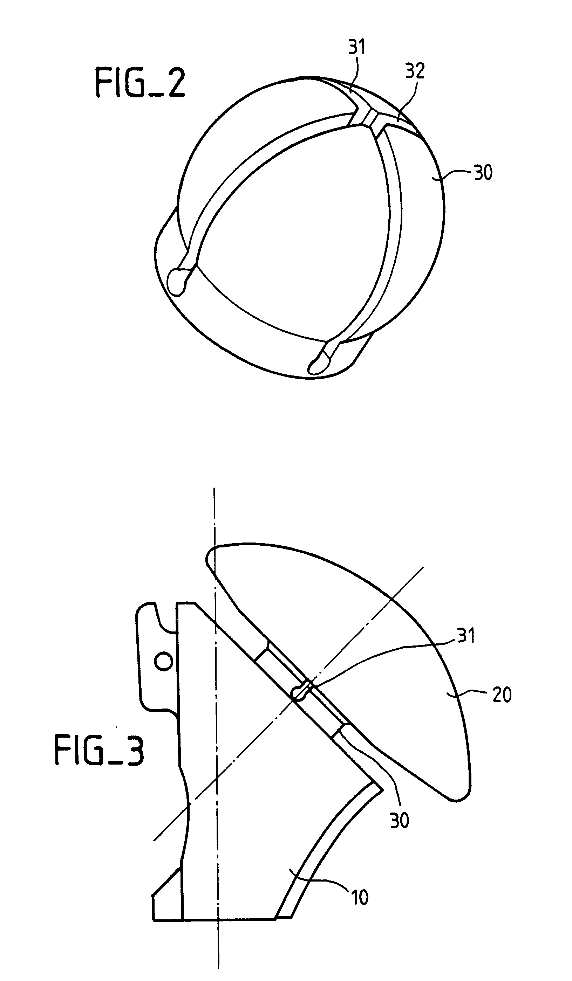

Le blocage en position de la calotte sphérique 20 sur la rotule 30 est obtenu à l'aide de moyens comprenant deux fentes médianes 31,32 orthogonales, aménagées dans la rotule sphérique 30, ainsi que le montre plus en détail la vue en perspective de la figure 2, l'ensemble constitué de la calotte 20 et de la rotule 30 étant illustré sur la figure 3.

Conformément au mode de réalisation de la figure 1, un alésage conique 33 axial débouchant dans les fentes médianes 31,32 reçoit un poussoir conique 50, lequel est commandé par une vis 51 de blocage, manoeuvrée de l'extérieur de la tige humérale 10, de manière à exercer une force de pression sur l'alésage conique 33 afin de provoquer une compression contre la cavité sphérique 21 par expansion latérale bloquée de ladite rotule 30.

## Revendications

1. Prothèse totale (1) d'épaule, comprenant :
- une tige humérale (10) destinée à être implantée à l'intérieur du canal huméral d'un patient,
- une calotte sphérique (20) de révolution autour d'un axe (D), destinée à coopérer avec la glène de l'épaule,
**caractérisée en ce que** ladite prothèse (1) comporte :
- des moyens de liaison de ladite calotte (20) à la tige humérale (10), comprenant une rotule sphérique (30) fixée à une extrémité proximale de ladite tige (10), et une cavité sphérique (21) aménagée dans ladite calotte (20) et formant logement pour ladite rotule (30), l'axe (D) de révolution de la calotte sphérique (20) étant déporté par rapport au centre (O) de ladite rotule sphérique (30), l'ensemble de la cavité (21) et de la rotule (30) constituant une articulation apte à faire varier l'orientation de la calotte sphérique (20) relativement à la tige (10) par rotation autour dudit centre (0) de la rotule,
- des moyens (31,32,33,50,51) de blocage en position de la calotte (20) sur la rotule sphérique (30).

2. Prothèse selon la revendication 1, **caractérisée en ce que** lesdits moyens de blocage comprennent, d'une part, au moins une fente médiane (31,32) et un alésage conique (33) axial débouchant dans ladite fente médiane (31,32), aménagés dans la rotule sphérique (30), et, d'autre part, un poussoir conique (50) disposé dans ledit alésage (33) et commandé par une vis (51) de blocage de manière à exercer une force de pression sur l'alésage conique (33) provoquant une compression contre la cavité sphérique (21) par expansion latérale bloquée de ladite rotule (30).

## Patentansprüche

1. Schulter-Vollprothese (1), die aufweist:
- einen Humerusschaft (10), der ins Innere des Humeruskanals eines Patienten implantiert werden soll,
- eine um eine Achse (D) drehsymmetrische, kugelförmige Kalotte (20), die mit der Gelenkpfanne der Schulter zusammenwirken soll,
**dadurch gekennzeichnet, daß** die Prothese (1) aufweist:
- Mittel zur Verbindung der Kalotte (20) mit dem Humerusschaft (10), die einen kugelförmigen Gelenkkopf (30), der an einem proximalen Ende des Schafts (10) befestigt ist, und einen kugelförmigen Hohlraum (21) aufweisen, der in der Kalotte (20) ausgebildet ist und einen Sitz für den Gelenkkopf (30) bildet, wobei die Drehachse (D) der kugelförmigen Kalotte (20) in bezug auf die Mitte (O) des kugelförmigen Gelenkkopfs (30) verschoben ist, wobei die Einheit aus Hohlraum (21) und Gelenkkopf (30) ein Gelenk bildet, das die Ausrichtung der kugelförmigen Kalotte (20) in bezug auf den Schaft (10) durch Drehung um die Mitte (O) des Gelenkkopfs verändern kann,
- Mittel (31, 32, 33, 50, 51) zur Positionsblockierung der Kalotte (20) auf dem kugelförmigen Gelenkkopf (30).

2. Prothese nach Anspruch 1, **dadurch gekennzeichnet, daß** die Blokkiermittel einerseits mindestens einen mittleren Schlitz (31, 32) und eine im mittleren Schlitz (31, 32) mündende, konische, axiale Bohrung (33) aufweisen, die im kugelförmigen Gelenkkopf (30) ausgebildet sind, und andererseits einen konischen Drücker (50) aufweisen, der in der Bohrung (33) angeordnet ist und durch eine Stellschraube (51) so gesteuert wird, daß er eine Druckkraft auf die konische Bohrung (33) ausübt, die durch blockierte seitliche Ausdehnung des Gelenkkopfs (30) einen Druck gegen den kugelförmigen Hohlraum (21) bewirkt.

## Claims

1. Total shoulder prosthesis (1) comprising
- a humeral stem (10) intended to be implanted inside the humeral marrow cavity of a patient,
- a spherical cup (20) revolving around an axis D, intended to work together with the shoulder socket,
**characterised in that** the said prosthesis (1) includes:
- means for linking the said cup (20) to the humeral stem (10), comprising a spherical joint ball (30) fixed to a proximal end of the said stem (10) and a spherical cavity (21) lodged in the said cup (20) and forming a seating for the said joint ball (30), the axis (D) of revolution of the spherical cup (20) being displaced relative to the centre (O) of the said spherical joint ball (30), the cavity (21) and joint ball (30) together constituting a joint able to vary the orientation of the spherical cup (20) relative to the stem (10) by rotation around the said centre (O) of the joint ball,
- means (31, 32, 33, 50, 51) for blocking the position of the cup (20) on the spherical joint ball (30).

2. Prosthesis according to Claim 1, **characterised in that** the said means of blocking comprise on the one hand at least one medial slot (31, 32) and an axial conical borehole (33) opening into the said median slot (31, 32) arranged in the spherical joint ball (30) and, on the other hand a conical push-rod (50) arranged in the said borehole (33) and operated by a blocking screw (51) such as to exert a pressure force on the conical borehole (33) that causes compression onto the spherical cavity (21) by blocked lateral expansion of the said joint ball (30).
